(19) Europäisches Patentamt
European Patent Office
Office européen des brevets

(11) Veröffentlichungsnummer: **0 004 317**
**B1**

(12) # EUROPÄISCHE PATENTSCHRIFT

(45) Veröffentlichungstag der Patentschrift: **06.01.82**

(21) Anmeldenummer: **79100698.4**

(22) Anmeldetag: **08.03.79**

(51) Int. Cl.³: **C 07 C 69/712**, C 07 C 59/70, A 01 N 39/02, A 01 N 43/40, A 01 N 37/34, C 07 C 121/75, C 07 C 79/46, C 07 C 103/78, C 07 C 153/09, C 07 C 149/20

(54) **Neue Phenoxy-alkancarbonsäurederivate, deren Herstellung, sie enthaltende herbizide Mittel und deren Verwendung.**

(30) Priorität: **17.03.78 CH 2932/78**

(43) Veröffentlichungstag der Anmeldung: **03.10.79 Patentblatt 79/20**

(45) Bekanntmachung des Hinweises auf die Patenterteilung: **06.01.82 Patentblatt 82/1**

(84) Benannte Vertragsstaaten: **BE CH DE FR GB IT NL**

(56) Entgegenhaltungen: **US-A-4 049 424**

(73) Patentinhaber: **CIBA-GEIGY AG, Patentabteilung Postfach, CH-4002 Basel (CH)**

(72) Erfinder: **Szczepanski, Henry, Dr., Waldshuterstrasse 55, CH-4310 Rheinfelden (CH)**
Erfinder: **Rohr, Otto, Dr., Kilbertweg 19, CH-4106 Therwil (CH)**
Erfinder: **Pissiotas, Georg, Dr., Breslauerstrasse 8, D-7850 Lörrach (DE)**
Erfinder: **Böhner, Beat, Dr., Hügelweg 3, CH-4102 Binningen (CH)**
Erfinder: **Rempfler, Hermann, Dr., Allschwilerweg 67, CH-4102 Binningen (CH)**

EP 0 004 317 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

# Neue Phenoxy-alkancarbonsäurederivate, deren Herstellung, sie enthaltende herbizide Mittel und deren Verwendung

Die vorliegende Erfindung betrifft neue, herbizid wirksame Phenoxy-alkancarbonsäurederivate, Verfahren zu ihrer Herstellung, ferner herbizide Mittel, die diese neuen Verbindungen als Wirkstoffe enthalten, sowie Verfahren zur selektiven Bekämpfung von Unkräutern in Kulturpflanzungen unter Verwendung der neuen Wirkstoffe oder der sie enthaltenden Mittel.

Die neuen Wirkstoffe entsprechen der Formel I

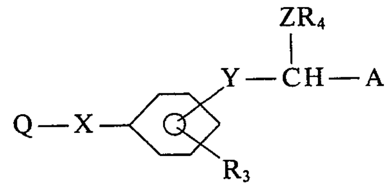

In dieser Formel bedeuten

Q ein Rest

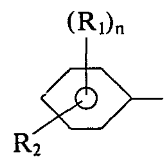

oder

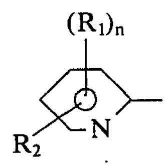

A die Cyanogruppe oder ein Rest-COB,
B ein Rest $-OR_5$, $-SR_6$, $-NR_7R_8$,
$R_1$ ein Halogenatom, n die Zahl 0, 1 oder 2,
$R_2$ ein Halogenatom oder der Trifluormethyl-, Nitro-, Cyano-, Carbamoyl- oder Thiocarbamoylrest,
$R_3$ Wasserstoff, Halogen, ein $C_1-C_4$ Alkyl-, der Nitro-, Cyano- oder Carbamoylrest,
$R_4$ $C_1-C_6$ Alkyl, $C_2-C_6$ Alkenyl, $C_2-C_6$ Alkinyl, Benzyl oder Phenyl, gegebenenfalls durch Halogen substituiert, $C_2-C_8$ Alkoxyalkyl, $C_3-C_{12}$ Cycloalkyl,
$R_5$ Wasserstoff oder das Kation einer Base

$$\frac{1}{m} M^{m\oplus}$$

M ein Alkali-, Erdalkali-Kation oder ein Fe, Cu-, Zn-, Mn-, Ni-Kation oder einen Ammonio-Rest

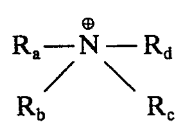

m als ganze Zahl 1, 2 oder 3 die Wertigkeit des Kations berücksichtigt, während $R_a$, $R_b$, $R_c$ und $R_d$ unabhängig voneinander Wasserstoff, Benzyl oder einen gegebenenfalls durch $-OH$, $-NH_2$ oder $C_1-C_4$ Alkoxy substituierten $C_1-C_4$ Alkylrest bedeuten, weiter bedeutet

$R_5$ und $R_6$ einen $C_1-C_{18}$ Alkyl-Rest, der unsubstituiert oder gegebenenfalls substituiert ist durch Halogen, Nitro, Cyano, $C_1-C_8$ Alkoxy, $C_2-C_8$-Alkoxyalkoxy, $C_3-C_6$ Alkenyloxy, $C_1-C_8$ Alkylthio, $C_2-C_8$ Alkanoyl, $C_2-C_8$ Acyloxy, $C_2-C_8$ Alkoxycarbonyl, Carbamoyl; Bis ($C_1-C_4$ alkyl)amino, Tris ($C_1-C_4$ alkyl)ammonio, $C_3-C_8$ Cycloalkyl, $C_3-C_8$ Cycloalkenyl, gegebenenfalls auch durch einen unsubstituierten oder seinerseits durch Halogen, $C_1-C_4$-Alkyl, $C_1-C_4$-Alkoxy ein- oder mehrfach substituierten Phenoxyrest oder 5 bis 6gliedrigen heterocyclischen Rest mit 1 bis 3 Heteroatomen;

- einen unsubst. oder ein- bis vierfach durch Halogen oder einmal durch Phenyl oder Methoxycarbonyl subst. $C_3-C_{18}$ Alkenylrest;
- einen $C_3-C_8$ Alkinyl-Rest;
- einen gegebenenfalls durch Halogen oder $C_1-C_4$-Alkyl substituierten $C_3-C_{12}$ Cycloalkyl-Rest;

- einen $C_3-C_8$ Cycloalkenyl-Rest;
- einen Phenylrest, der unsubstituiert oder durch Halogen, $C_1-C_4$-Alkyl, $C_1-C_4$ Alkoxy, $C_1-C_4$-Alkylthio, $NO_2$, $CF_3$, COOH, CN, OH, $SO_3H$, $NH_2$ oder $-NH(C_1-C_4$ Alkyl) oder $-N(C_1-C_4$ Alkyl$)_2$ ein- oder mehrfach substituiert ist;
- einen 5- bis 6gliedrigen heterocyclischen Ring mit 1 bis 3 Heteroatomen;

$R_7$ und $R_8$ je Wasserstoff oder einen niederen Alkylrest, oder eines davon auch einen verzweigten Alkenyl- oder Alkinylrest oder den Phenyl-, Benzyl-, Cyclohexyl- oder den Methoxyrest,
$R_7$ und $R_8$ zusammen mit dem Stickstoffatom, an das sie gebunden sind, auch einen heterocyclischen Rest bilden,
X, Y und Z je ein Sauerstoff- oder Schwefelatom bedeuten.

Die Alkylreste in dieser Formel können verzweigt oder unverzweigt sein und enthalten die angegebene Anzahl Kohlenstoffatome.

Die neuen Wirkstoffe der Formel I gemäß vorliegender Erfindung besitzen Herbizidwirkung bei pre- und post-emergenter Anwendung und können in mono- und dikotylen Kulturen als Unkrautmittel eingesetzt werden. Speziell zu erwähnen ist ihre Wirkung gegen das Unkraut Dachtrespe (bromus tectorum).

Ferner besitzen sie günstige wachstumsregulierende Effekte (Wuchshemmung). Insbesondere hemmen sie das Wachstum von dicotylen Pflanzen. Beispiele für die nutzbringende Anwendung der erfindungsgemäßen Verbindungen sind z. B.

- die Reduktion des vegetativen Wachstums bei Soja und ähnlichen Leguminosen, was zu einer Ertragssteigerung dieser Kultur führt;
- die Hemmung des unerwünschten Wachstums von Geiztrieben bei Tabak, dessen Haupttrieb man geschnitten hat, was der Ausbildung größerer und schönerer Blätter zugute kommt;
- die Hemmung des Wachstums von Gras und dikotyledonen Pflanzen, wie Obstbäume, Zierbäume, Gebüsche und Hecken, zwecks Einsparung an Schnittarbeit.
- Die Austrocknung und Entblätterung von Pflanzen z. B. Kartoffeln und Baumwolle kurz bevor deren Ernte.

Die Verbindungen vorliegender Erfindung sind wenig giftig für Warmblüter und deren Applikation wirft keine Probleme auf. Die Aufwandmenge liegt zwischen 0,1 und 5 kg pro Hektar.

Die Herstellung der neuen Verbindungen der Formel I erfolgt nach an sich bekannten Methoden, zum Beispiel gemäß den folgenden Synthesewegen.

Ein entsprechend substituiertes Phenol oder Thiophenol der Formel II

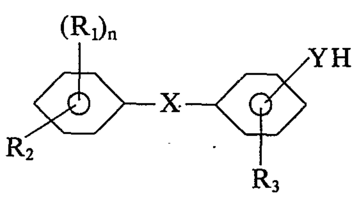

(II)

worin $R_1$, n, $R_2$, $R_3$, X und Y die unter Formel I gegebene Bedeutung haben, wird in einem inerten Lösungs- oder Verdünnungsmittel in Gegenwart einer Base als säurebindendes Mittel mit einem in 2-Stellung durch $-ZR_4$ substituierten Derivat einer 2-Halogenessigsäure der Formel III umgesetzt,

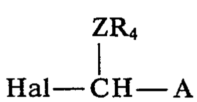

(III)

worin A, $R_4$ und Z die unter Formel I gegebene Bedeutung haben, während »Hal« ein Halogenatom, vorzugsweise Chlor- oder Brom bedeuten.

Man gelangt ebenfalls zu den Verbindungen der Formel I indem man ein entsprechend substituiertes Derivat einer 2-Halogenessigsäure entsprechend der Formel IV

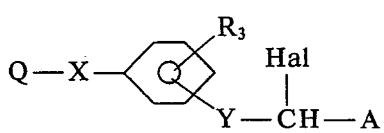

(IV)

worin A, Q, $R_3$, X und Y die unter Formel I gegebene Bedeutung haben, während »Hal« ein Halogenatom, vorzugsweise Chlor oder Brom bedeutet, in einem inerten Lösungs- oder Verdünnungsmittel in Gegenwart einer Base als säurebindendes Mittel, mit einem Alkohol oder Thiol der Formel V umsetzt,

$$H—Z—R_4 \qquad (V)$$

worin $R_4$ und Z die unter Formel I gegebene Bedeutung haben.

Die genannten Umsetzungen können in An- oder Abwesenheit von gegenüber den Reaktionsteilnehmern inerten Lösungs- oder Verdünnungsmitteln durchgeführt werden, bevorzugt sind polare organische Lösungsmittel wie Methyläthylketon, Dimethylformamid, Dimethylsulfoxid etc. Die Reaktionstemperaturen liegen zwischen 0 und 200° und die Reaktionsdauer beträgt je nach Ausgangsstoff, gewählter Umsetzungstemperaturen und Lösungsmittel zwischen 1 Stunde und mehreren Tagen. Man arbeitet in der Regel bei Normaldruck. Als Basen (Kondensationsmittel) für die Umsetzung kommen die üblichen, wie z. B. KOH, $NaOCH_3$, $NaHCO_3$, $K_2CO_3$, Kalium-tert.butylat etc. aber auch organische Basen wie Triäthylamin etc. in Betracht.

Die Verbindungen der Formel I sind neu. Strukturähnliche 2-Phenoxy-2-alkoxy-essigsäureamide mit herbizider Wirkung sind in den US-Patenten No. 4 049 423, 4 049 424 sowie 4 050 923 beschrieben worden.

Die Ausgangsstoffe der Formeln II bis V sind teilweise bekannt. Noch nicht beschriebene Ausgangsstoffe dieser Formeln lassen sich nach üblichen Verfahren und Techniken leicht herstellen. Phenoxyphenole der Formel II können beispielsweise gemäß den in J. Am. Chem. Soc. 61, 2702 (1939) oder in Chem. Abstract 52 9006 b (1958) beschriebenen Methoden hergestellt werden.

In den nachfolgenden Beispielen wird die Herstellung von Phenoxy-alkancarbonsäureestern der Formel I veranschaulicht. Weitere in entsprechender Weise hergestellte Wirkstoffe sind in den anschließenden Tabellen aufgeführt. Temperaturen sind in Celsiusgraden angegeben. Teile und Prozentangaben beziehen sich auf das Gewicht.

## Beispiel 1

2-[4-(4′-Trifluormethylphenoxy)-phenoxy]-2-methoxy-essigsäure-methylester

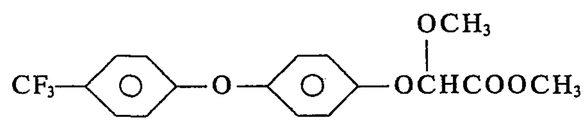

Zu einer Lösung von 33,3 g (0,3 Mol) Kaliumtertiärbutylat in 300 ml tert.Butanol gibt man zuerst 76,2 g (0,3 Mol) 4-(4′-Trifluormethylphenoxy)-phenol und dann tropfenweise 54,9 g (0,3 2-Brom-2-methoxy-essigsäure-methylester. Nach 3stündigem Rühren bei 35° wird das Reaktionsgemisch auf 1200 ml Wasser gegossen und dann zweimal mit je 300 ml Chloroform extrahiert. Die vereinigten Extrakte werden dreimal mit je 300 ml gesättigter wäßriger NaCl-Lösung gewaschen, über Magnesiumsulfat getrocknet und eingedampft. Nach Abdestillieren des Lösungsmittels verbleiben 97,3 g (0,274 Mol) des Titelproduktes als Öl zurück, das einen Brechungsindex von n = 1,5119 hat und durch das NMR Spektrum identifiziert wurde.

## Beispiel 2

2-Methoxy-2-[3-(3′,5′-Dichlor-pyridyl-2′-oxy)-6-chlorphenoxy]-essigsäure-methylester

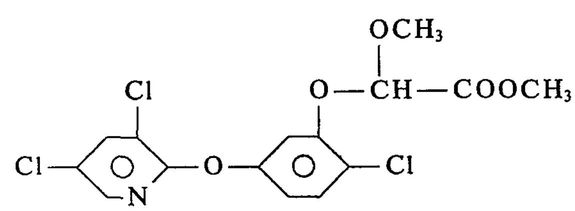

Zu einer Lösung von 6,2 g (0,055 Mol) Kalium-tert. butylat in 140 ml tert.Butanol werden 14,5 g (0,05 Mol) 3-(3′,5′-Dichlor-pyridyl-2′-oxy)-6-chlorphenol gegeben. Bei 40° werden 10 g (0,055 Mol) 2-Brom-2-methoxy-essigsäure-methylester zugetropft. Das Reaktionsgemisch wird 6 Stunden bei 25° gerührt. Das Kaliumbromid wird dann abfiltriert und das Filtrat eingedampft. Das verbleibende Öl wird in Äther aufgenommen und mit Wasser extrahiert. Die Ätherphase wird mit Magnesiumsulfat getrocknet und eingedampft. Nach dem Trocknen am Hochvakuum erhält man 14 g (71% der Theorie) der Titelverbindung mit dem Brechungsindex $n_D^{30}$ 1,5675.

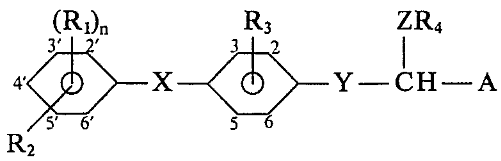

Tabelle 1

| No. | 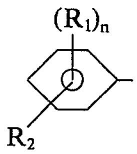 | X | 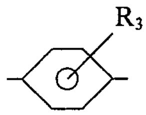 | Y | $ZR_4$ | A | physikalische Konstante |
|---|---|---|---|---|---|---|---|
| 1 | 4'$CF_3$ | O | H | O | $OCH_3$ | $-COOCH_3$ | $n_D^{22}$ 1,5199 |
| 2 | 4'$CF_3$ | O | H | O | $OCH_3$ | $-COOH$ | |
| 3 | 4'$CF_3$ | O | H | O | $OCH_3$ | $-COOC_2H_5$ | |
| 4 | 4'$CF_3$ | O | H | O | $OCH_3$ | $-COO_nC_3H_7$ | |
| 5 | 4'$CF_3$ | O | H | O | $OCH_3$ | $-COOiC_3H_7$ | |
| 6 | 4'$CF_3$ | O | H | O | $OCH_3$ | $-COO_sC_4H_9$ | |
| 7 | 4'$CF_3$ | O | H | O | $OCH_3$ | $-COOiC_4H_9$ | |
| 8 | 4'$CF_3$ | O | H | O | $OCH_3$ | $-COO-$ 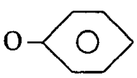 | |
| 9 | 4'$CF_3$ | O | H | O | $OCH_3$ | $-COOCH_2-$ 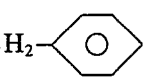 | |
| 10 | 4'$CF_3$ | O | H | O | $OCH_3$ | $-COO-$ 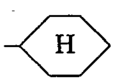 | |
| 11 | 4'$CF_3$ | O | H | O | $OCH_3$ | $-COOCH_2-CH=CH_2$ | |
| 12 | 4'$CF_3$ | O | H | O | $OCH_3$ | $-COSH$ | |
| 13 | 4'$CF_3$ | O | H | O | $OCH_3$ | $-COSCH_3$ | |
| 14 | 4'$CF_3$ | O | H | O | $OCH_3$ | $-COSCH_2-CH=CH_2$ | |
| 15 | 4'$CF_3$ | O | H | O | $OCH_3$ | $-COSCH_2-$ 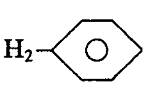 | |
| 16 | 4'$CF_3$ | O | H | O | $OCH_3$ | $-CONHCH_2-$ 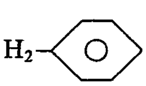 | |
| 17 | 4'$CF_3$ | O | H | O | $OCH_3$ | $-CONHC(CH_3)_2C\equiv CH$ | |
| 18 | 4'$CF_3$ | O | H | O | $OCH_3$ | $-CONHC(C_2H_5)_2C\equiv CH$ | |
| 19 | 4'$CF_3$ | O | H | O | $OCH_3$ | $-COOC_2H_4OCH_3$ | |
| 20 | 4'$CF_3$ | O | H | O | $OCH_3$ | $-CN$ | |
| 21 | 4'$CF_3$ | O | H | O | $OC_2H_5$ | $-COOCH_3$ | |

Fortsetzung

| No. | 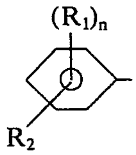 | X | 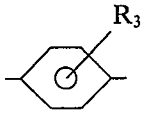 | Y | $ZR_4$ | A | physikalische Konstante |
|---|---|---|---|---|---|---|---|
| 22 | 2'Cl, 4'$CF_3$ | O | H | O | $OCH_3$ | $-COOCH_3$ | $n_D^{20}$ 1,5206 |
| 23 | 2'Cl, 4'$CF_3$ | O | H | O | $OCH_3$ | $-COOH$ | |
| 24 | 2'Cl, 4'$CF_3$ | O | H | O | $OCH_3$ | $-COOC_2H_5$ | |
| 25 | 2'Cl, 4'$CF_3$ | O | H | O | $OCH_3$ | $-COO_nC_3H_7$ | |
| 26 | 2'Cl, 4'$CF_3$ | O | H | O | $OCH_3$ | $-COOiC_3H_7$ | |
| 27 | 2'Cl, 4'$CF_3$ | O | H | O | $OCH_3$ | $-COOC_4H_9$ | |
| 28 | 2'Cl, 4'$CF_3$ | O | H | O | $OCH_3$ | $-COOiC_4H_9$ | |
| 29 | 2'Cl, 4'$CF_3$ | O | H | O | $OCH_3$ | $-COOCH_2-$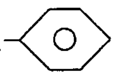 | |
| 30 | 2'Cl, 4'$CF_3$ | O | H | O | $OCH_3$ | $-COO-$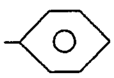 | |
| 31 | 2'Cl, 4'$CF_3$ | O | H | O | $OCH_3$ | $-COO-$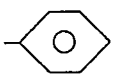 | |
| 32 | 2'Cl, 4'$CF_3$ | O | H | O | $OCH_3$ | $-COOC_2H_4OCH_3$ | |
| 33 | 2'Cl, 4'$CF_3$ | O | H | O | $OCH_3$ | $-COOCH_2-CH=CH_2$ | |
| 34 | 2'Cl, 4'$CF_3$ | O | H | O | $OCH_3$ | $-COOCH_2-CH\equiv CN$ | |
| 35 | 2'Cl, 4'$CF_3$ | O | H | O | $OCH_3$ | $-COSCH_3$ | |
| 36 | 2'Cl, 4'$CF_3$ | O | H | O | $OCH_3$ | $-COSC_2H_5$ | |
| 37 | 2'Cl, 4'$CF_3$ | O | H | O | $OCH_3$ | $-COSCH_2-CH=CH_2$ | |
| 38 | 2'Cl, 4'$CF_3$ | O | H | O | $OCH_3$ | $-COSCH_2-$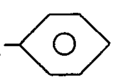 | |
| 39 | 2'Cl, 4'$CF_3$ | O | H | O | $OCH_3$ | $-CONHCH_2-$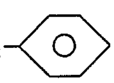 | |
| 40 | 2'Cl, 4'$CF_3$ | O | H | O | $OCH_3$ | $-CONHC(CH_3)_2C\equiv CH$ | |
| 41 | 2'Cl, 4'$CF_3$ | O | H | O | $OCH_3$ | $-CONHC(C_2H_5)_2C\equiv CH$ | |
| 42 | 2'Cl, 4'$CF_3$ | O | H | O | $OCH_3$ | $-CN$ | |
| 43 | 2'Cl, 4'$CF_3$ | O | H | O | $OC_2H_5$ | $-COOCH_3$ | |
| 44 | 2'Cl, 4'$CF_3$ | O | H | O | $OCH_3$ | $-CON(CH_3)_2$ | |
| 45 | 2'Cl, 4'$CF_3$ | O | H | O | $OCH_3$ | $-COOCH_3$ | |

Fortsetzung

| No. | 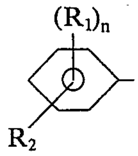 | X | 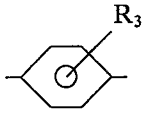 | Y | $ZR_4$ | A | physikalische Konstante |
|---|---|---|---|---|---|---|---|
| 46 | 2'$NO_2$, 4'Cl | O | H | O | $OCH_3$ | $-COOCH_3$ | Smp. 62–65° |
| 47 | 2'CN, 4'Cl | O | H | O | $OCH_3$ | $-COOCH_3$ | $n_D^{20}$ 1,5496 |
| 48 | 2'$CSNH_2$, 4'Cl | O | H | O | $OCH_3$ | $-COOCH_3$ | |
| 49 | 4'Cl | O | H | O | $OCH_3$ | $-COOCH_3$ | $n_D^{20}$ 1,5550 |
| 50 | 2'Cl, 4'Cl | O | H | O | $OCH_3$ | $-COOCH_3$ | $n_D^{20}$ 1,5641 |

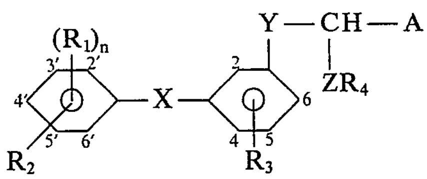

Tabelle 2

| No. | 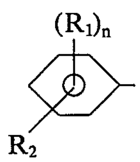 | X | 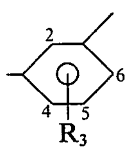 | Y | $ZR_4$ | A | physikalische Konstante |
|---|---|---|---|---|---|---|---|
| 1 | 2'Cl, 4'Cl | O | 6 $NO_2$ | O | $OCH_3$ | $-COOCH_3$ | |
| 2 | 2'Cl, 4'Cl | O | 6 CN | O | $OCH_3$ | $-COOCH_3$ | |
| 3 | 2'Cl, 4'Cl | O | 6 Cl | O | $OCH_3$ | $-COOCH_3$ | $n_D^{20}$ 1,5659 |
| 4 | 2'$NO_2$, 4'Cl | O | 6 Cl | O | $OCH_3$ | $-COOCH_3$ | $n_D^{20}$ 1,5598 |
| 5 | 2'$NO_2$, 4'Cl | O | H | O | $OCH_3$ | $-COOCH_3$ | |
| 6 | 2'Cl, 4'Br | O | 6 Cl | O | $OCH_3$ | $-COOCH_3$ | $n_D^{20}$ 1,5710 |
| 7 | 2'CN, 4'Cl | O | H | O | $OCH_3$ | $-COOCH_3$ | $n_D^{20}$ 1,5645 |
| 8 | 2'CN, 4'Cl | O | 6 $NO_2$ | O | $OCH_3$ | $-COOCH_3$ | $n_D^{20}$ 1,5598 |
| 9 | 2'Cl, 4'$CF_3$ | O | 6 Cl | O | $OCH_3$ | $-COOCH_3$ | $n_D^{20}$ 1,5320 |
| 10 | 2'Cl, 4'$CF_3$ | O | 6 $NO_2$ | O | $OCH_3$ | $-COOCH_3$ | $n_D^{23}$ 1,5268 |
| 11 | 2'Cl, 4'$CF_3$ | O | 6 Cl | O | $SCH_3$ | $-COOCH_3$ | $n_D^{20}$ 1,5433 |
| 12 | 2'Br, 4'Cl | O | 6 Cl | O | $OCH_3$ | $-COOCH_3$ | $n_D^{20}$ 1,5707 |
| 13 | 2'Cl, 4'$CF_3$ | O | 6 Cl | O | $SC_2H_5$ | $-COOCH_3$ | $n_D^{20}$ 1,5402 |

Fortsetzung

| No. | 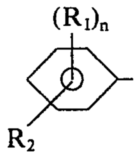 | X | 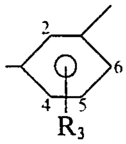 | Y | $ZR_4$ | A | physikalische Konstante |
|---|---|---|---|---|---|---|---|
| 14 | 2'Cl, 4'$CF_3$ | O | 6 Cl | O | $OCH_3$ | $-COOC_2H_5$ | $n_D^{20}$ 1,5172 |
| 15 | 2'Cl, 4'$CF_3$ | O | 6 Cl | O | $OCH_3$ | $-COOH$ | $n_D^{20}$ 1,5348 |
| 16 | 2'Cl, 4'$CF_3$ | O | 6 $NO_2$ | O | $OCH_3$ | $-COOH$ | $n_D^{20}$ 1,5402 |
| 17 | 2'Cl, 4'$CF_3$ | O | 6 $NO_2$ | O | $OCH_3$ | $-COOC_2H_5$ | $n_D^{20}$ 1,5314 |
| 18 | 2'Cl, 4'$CF_3$ | O | 6 Cl | O | $OCH_3$ | $-COO_tC_4H_9$ | $n_D^{20}$ 1,5252 |
| 19 | 2'Cl, 4'$CF_3$ | O | 6 $NO_2$ | O | $SCH_3$ | $-COOCH_3$ | $n_D^{20}$ 1,5510 |
| 20 | 2'Cl, 4'$CF_3$ | O | 6 $NO_2$ | O | $SC_2H_5$ | $-COOCH_3$ | $n_D^{20}$ 1,5427 |
| 21 | 2'CN, 4'Cl | O | 6 $NO_2$ | O | $OCH_3$ | $-COOCH_3$ | |
| 22 | 2'Cl, 4'$CF_3$ | O | 6 Cl | O | $OCH_3$ | $-COOC_2H_4OCH_3$ | |
| 23 | 2'Cl, 4'$CF_3$ | O | 6 Cl | O | $SCH_3$ | $-COOCH_2CH=CH_2$ | |
| 24 | 2'Cl, 4'$CF_3$ | O | 6 $NO_2$ | O | $SCH_3$ | $-COO-$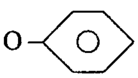 | |
| 25 | 2'Cl, 4'$CF_3$ | O | 6 $NO_2$ | O | $SCH_3$ | $-COO-$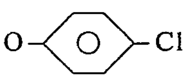 | |
| 26 | 2'Cl, 4'$CF_3$ | O | 6 Cl | O | $SCH_3$ | $-COOC_2H_4SCH_3$ | |
| 27 | 2'Cl, 4'$CF_3$ | O | 6 Cl | O | $SCH_3$ | $-COSCH_2CH=CH_2$ | |
| 28 | 2'Cl, 4'$CF_3$ | O | 6 $NO_2$ | O | $OCH_3$ | $-COS-$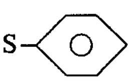 | |
| 29 | 2'Cl, 4'$CF_3$ | O | 6 $NO_2$ | O | $OCH_3$ | $-COS-$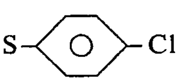 | |
| 30 | 2'Cl, 4'$CF_3$ | O | 6 $NO_2$ | O | $OCH_3$ | $-COC_2H_4OC_2H_4OC_2H_5$ | |
| 31 | 2'Cl, 4'$CF_3$ | O | 6 Cl | O | $OCH_3$ | $-COOC_2H_4OC_2H_4OC_2H_5$ | |
| 32 | 2'Cl, 4'$CF_3$ | O | 6 Cl | O | $OCH_3$ | $-COOC_2H_4Cl$ | |
| 33 | 2'Cl, 4'$CF_3$ | O | 6 $NO_2$ | O | $OCH_3$ | $-COOC_2H_4Cl$ | |
| 34 | 2'Cl, 4'$CF_3$ | O | 6 Cl | O | $SCH_3$ | $-COOC_2H_4Cl$ | |
| 35 | 2'Cl, 4'$CF_3$ | O | 6 $NO_2$ | O | $OCH_3$ | $-COOC_2H_4CN$ | |
| 36 | 2'Cl, 4'$CF_3$ | O | 6 Cl | O | $OCH_3$ | $-COOC_2H_4CN$ | |
| 37 | 2'Cl, 4'$CF_3$ | O | 6 Cl | O | $OCH_3$ | $-COOCH_2CF_3$ | |

Fortsetzung

| No. | 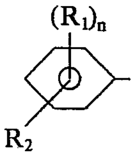 | X | 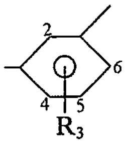 | Y | $ZR_4$ | A | physikalische Konstante |
|---|---|---|---|---|---|---|---|
| 38 | 2'Cl, 4'$CF_3$ | O | 6 $NO_2$ | O | $OCH_3$ | $-COOCH_2CF_3$ | |
| 39 | 2'Cl, 4'$CF_3$ | O | 6 Cl | O | O- 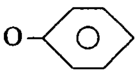 | $-COOCH_3$ | $n_D^{21}$ 1,5459 |
| 40 | 2'Cl, 4'$CF_3$ | O | 6 Cl | O | S- 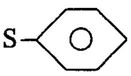 | $-COOCH_3$ | Smp. 122° |
| 41 | 2'Cl, 4'Cl | O | 6 Cl | O | S- 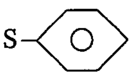 | $-COOCH_3$ | Smp. 102° |
| 42 | 2'Cl, 4'$CF_3$ | O | 6 Cl | O | S- 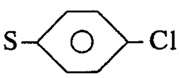 | $-COOCH_3$ | Smp. 95° |
| 43 | 2'Cl, 4'Cl | O | 6 Cl | O | S- 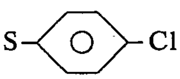 | $-COOCH_3$ | Smp. 85° |
| 44 | 2'Cl, 4'Cl | O | 6 Cl | O | O- 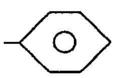 | $-COOCH_3$ | |
| 45 | 2'Cl, 4'$CF_3$ | O | 6 $NO_2$ | S | $OCH_3$ | $-COOCH_3$ | |
| 46 | 2'Cl, 4'$CF_3$ | O | 6 $NO_2$ | S | $SCH_2$ | $-COOCH_3$ | |
| 47 | 2'Cl, 4'$CF_3$ | O | 6 $NO_2$ | S | $SC_2H_5$ | $-COOC_2H_5$ | |
| 48 | 2'Cl, 4'$CF_3$ | O | 6 $NO_2$ | S | $SC_2H_5$ | $-COOCH_3$ | |
| 49 | 2'Cl, 4'$CF_3$ | O | 6 $NO_2$ | O | $O_sC_4H_9$ | $-COOCH_3$ | $n_D^{21}$ 1,5188 |
| 50 | 2'Cl, 4'$CF_3$ | O | 6 Cl | O | $OC_2H_5$ | $-COOC_2H_5$ | $n_D^{21}$ 1,5145 |
| 51 | 2'Cl, 4'$CF_3$ | O | 6 Cl | O | $O_nC_3H_7$ | $-CO_nC_3H_7$ | $n_D^{21}$ 1,5090 |
| 52 | 2'Cl, 4'$CF_3$ | O | 6 Cl | O | $O_sC_4H_9$ | $-COO_sC_4H_9$ | $n_D^{21}$ 1,5069 |
| 53 | 2'Cl, 4'$CF_3$ | O | 6 Cl | O | $O_sC_4H_9$ | $-COOCH_3$ | $n_D^{21}$ 1,5060 |
| 54 | 2'Cl, 4'$CF_3$ | O | 6 $NO_2$ | O | $OC_2H_5$ | $-COOCH_3$ | $n_D^{22}$ 1,5261 |
| 55 | 2'Cl, 4'$CF_3$ | O | 6 $NO_2$ | O | $O_sC_4H_9$ | $-COO_sC_4H_9$ | $n_D^{21}$ 1,5179 |
| 56 | 2'Cl, 4'$CF_3$ | O | 6 $NO_2$ | O | $O_nC_3H_7$ | $-COO_nC_3H_7$ | $n_D^{21}$ 1,5202 |
| 57 | 2'Cl, 4'$CF_3$ | O | 6 $NO_2$ | O | $OCH_3$ | $-CONH-$ 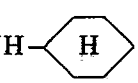 | |
| 58 | 2'Cl, 4'$CF_3$ | O | 6 $NO_2$ | O | $OCH_3$ | $-COOCH_2{\equiv}CH$ | |
| 59 | 2'Cl, 4'$CF_3$ | O | 6 $NO_2$ | O | $OCH_3$ | $-CONHCH_2-$ 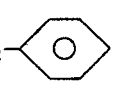 | |

Fortsetzung

| No. | 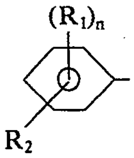 | X | 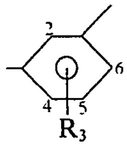 | Y | ZR4 | A | physikalische Konstante |
|---|---|---|---|---|---|---|---|
| 60 | 2'Cl, 4'$CF_3$ | O | 6 $NO_2$ | O | $OCH_3$ | $-CON(CH_3)_2$ | |
| 61 | 2'Cl, 4'$CF_3$ | O | 6 Cl | O | $SCH_3$ | $-COOCH_3$ | |
| 62 | 2'Cl, 4'$CF_3$ | O | 6 Cl | O | $SC_2H_5$ | $-COOCH_3$ | |
| 63 | 2'Cl, 4'$CF_3$ | O | 6 Cl | O | $OCH_3$ | $-CONHC(CH_3)C{\equiv}CH$ | |
| 64 | 2'Cl, 4'$CF_3$ | O | 6 $NO_2$ | O | $OCH_3$ | $-CON(CH_3)OCH_3$ | |
| 65 | 2'Cl, 4'$CF_3$ | O | 6 $NO_2$ | O | $OCH_3$ | $-CONHC(C_2H_5)_2C{\equiv}CH$ | |
| 66 | 2'Cl, 4'Cl | O | 6 Cl | O | $OCH_3$ | $-CON(CH_3)_2$ | |
| 67 | 2'Cl, 4'$CF_3$ | O | 6 $NO_2$ | O | $SCH_3$ | $-COOCH_2CH{=}CH_2$ | |
| 68 | 2'Cl, 4'$CF_3$ | O | 6 Cl | O | $OCH_2CH{=}CH_2$ | $-COOCH_3$ | |

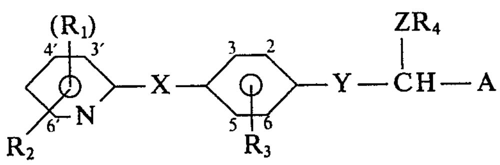

Tabelle 3

| No. | 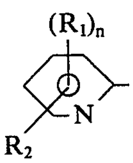 | X | 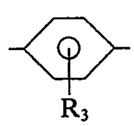 | Y | $ZR_4$ | A | physikalische Konstante |
|---|---|---|---|---|---|---|---|
| 1 | 3'Cl, 5'Cl | O | H | O | $OCH_3$ | $-COOCH_3$ | $n_D^{30}$ 1,5633 |
| 2 | 3'Cl, 5'Cl | O | H | O | $OCH_3$ | $-COOC_2H_5$ | |
| 3 | 3'Cl, 5'Cl | O | H | O | $OCH_3$ | $-COOH$ | |
| 4 | 3'Cl, 5'Cl | O | H | O | $OCH_3$ | $-CN$ | |
| 5 | 3'Cl, 5'Cl | O | H | O | $SCH_3$ | $-COOCH_3$ | |
| 6 | 3'Br, 5'Br | O | H | O | $OCH_3$ | $-COOCH_3$ | |
| 7 | 3'Br, 5'Cl | O | H | O | $OCH_3$ | $-COOCH_3$ | |
| 8 | 3'CN, 5'Cl | O | H | O | $OCH_3$ | $-COOCH_3$ | |
| 9 | 3'Cl, 5'CN | O | H | O | $OCH_3$ | $-COOCH_3$ | |

Fortsetzung

| No. | 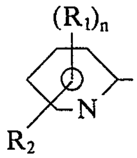 | X | 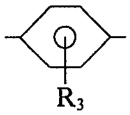 | Y | $ZR_4$ | A | physikalische Konstante |
|---|---|---|---|---|---|---|---|
| 10 | 3'Cl, 5'Br | O | H | O | $OCH_3$ | $-COOCH_3$ | |
| 11 | 3'Br, 5'Br | O | H | O | $OCH_3$ | $-COOC_2H_5$ | |
| 12 | 3'Cl, 5'Cl | O | H | O | $OCH_3$ | $-COOCH_2-CH=CH_2$ | |
| 13 | 3'Cl, 5'Cl | O | H | O | $OCH_3$ | $-CON(CH_3)_2$ | |
| 14 | 3'Cl, 5'Cl | O | H | O | $SCH_3$ | $-CONH-$ 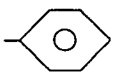 | |

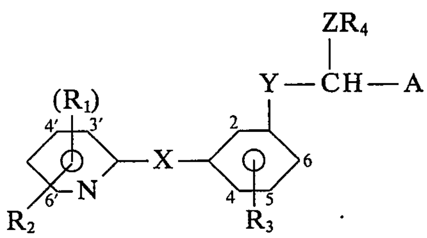

Tabelle 4

| No. | 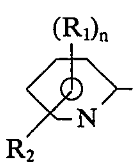 | X | 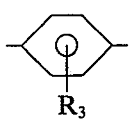 | Y | $ZR_4$ | A | physikalische Konstante |
|---|---|---|---|---|---|---|---|
| 1 | 3'Cl, 5'Cl | O | 6 Cl | O | $OCH_3$ | $COOCH_3$ | $n_D^{30}$ 1,5675 |
| 2 | 3'Cl, 5'Cl | O | 6 Cl | O | $OCH_3$ | COOH | |
| 3 | 3'Cl, 5'Cl | O | 6 Cl | O | $OCH_3$ | $COOC_2H_5$ | |
| 4 | 3'Cl, 5'Cl | O | 6 Cl | O | $OCH_3$ | $COO_nC_3H_7$ | |
| 5 | 3'Cl, 5'Cl | O | 6 Cl | O | $OCH_3$ | $COO_iC_3H_7$ | |
| 6 | 3'Cl, 5'Cl | O | 6 Cl | O | $OCH_3$ | COO- 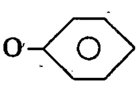 | |
| 7 | 3'Cl, 5'Cl | O | 6 Cl | O | $OCH_3$ | $COOCH_2-$ 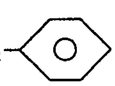 | |
| 8 | 3'Cl, 5'Cl | O | 6 Cl | O | $OCH_3$ | COO- 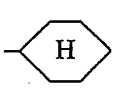 | |
| 9 | 3'Cl, 5'Cl | O | 6 Cl | O | $OCH_3$ | $COOCH_2CH=CH_2$ | |
| 10 | 3'Cl, 5'Cl | O | 6 Cl | O | $OCH_3$ | $COS_iC_3H_7$ | |

Fortsetzung

| No. | 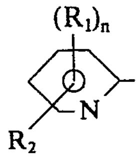 | X | 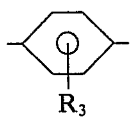 | Y | $ZR_4$ | A | physikalische Konstante |
|---|---|---|---|---|---|---|---|
| 11 | 3'Cl, 5'Cl | O | 6 Cl | O | $OCH_3$ | $COSCH_3$ | |
| 12 | 3'Cl, 5'Cl | O | 6 Cl | O | $OCH_3$ | $COSCH_2CH=CH_2$ | |
| 13 | 3'Cl, 5'Cl | O | 6 Cl | O | $OCH_3$ | $COSCH_2-$ 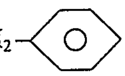 | |
| 14 | 3'Cl, 5'Cl | O | 6 Cl | O | $OCH_3$ | $CONHCH_2-$ 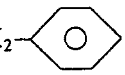 | |
| 15 | 3'Cl, 5'Cl | O | 6 Cl | O | $OCH_3$ | CN | |
| 16 | 3'Cl, 5'Cl | O | 6 Cl | O | $OCH_3$ | $CONHC(CH_3)_2C\equiv CH$ | |
| 17 | 3'Cl, 5'Cl | O | 6 Cl | O | $OCH_3$ | $CONHC(C_2H_5)_2C\equiv CH$ | |
| 18 | 3'Cl, 5'Cl | O | 6 Br | O | $OCH_3$ | $-COOCH_3$ | $n_D^{20}$ 1,5815 |
| 19 | 3'Cl, 5'Cl | O | 6 $NO_2$ | O | $OCH_3$ | $-COOCH_3$ | $n_D^{20}$ 1,5830 |
| 20 | 3'Cl, 5'Cl | O | 6 Br | O | $OCH_3$ | $-CON(CH_3)_2$ | |
| 21 | 3'Br, 5'Br | O | 6 Br | O | $OCH_3$ | $-COOCH_3$ | |
| 22 | 3'Cl, 5'Cl | O | 6 Cl | O | $SCH_3$ | $-COOCH_3$ | |
| 23 | 3'Cl, 5'Cl | O | 6 Br | O | $SCH_3$ | $-COOCH_3$ | |
| 24 | 3'Cl, 5'Cl | O | 6 $NO_2$ | O | $SCH_3$ | $-COOCH_3$ | |
| 25 | 3'CN, 5'Cl | O | 6 Cl | O | $SCH_3$ | $-COOCH_3$ | |
| 26 | 3'Cl, 5'Br | O | 6 Cl | O | $OCH_3$ | $-COOCH_3$ | |
| 27 | H, 5'Cl | O | 6 Cl | O | $OCH_3$ | $-COOCH_3$ | |

Die neuen Wirkstoffe der Formel I sind stabile Verbindungen, welche in üblichen organischen Lösungsmitteln, wie Alkoholen, Äthern, Ketonen, Dimethylformamid, Dimethylsulfoxyd etc. löslich sind.

Die Herstellung erfindungsgemäßer Mittel erfolgt in an sich bekannter Weise durch inniges Vermischen und Vermahlen von Wirkstoffen der allgemeinen Formel I mit geeigneten Trägerstoffen und/oder Verteilungsmitteln, gegebenenfalls unter Zusatz von gegenüber den Wirkstoffen inerten Antischaum-, Netz-, Dispersions- und/oder Lösungsmitteln. Die Wirkstoffe können in den folgenden Aufarbeitungsformen vorliegen und angewendet werden:

Feste Aufarbeitungsformen:
Stäubemittel, Streumittel, Granulate, Umhüllungsgranulate, Imprägniergranulate und Homogengranulate;
In Wasser dispergierbare Wirkstoffkonzentrate:
Spritzpulver (wettable powder), Pasten, Emulsionen; Emulsionskonzentrate
Flüssige Aufarbeitungsformen:
Lösungen

Die Wirkstoffkonzentrationen betragen in den erfindungsgemäßen Mitteln 1 bis 80 Gewichtsprozent und können gegebenenfalls bei der Anwendung auch in geringen Konzentrationen wie etwa 0,05 bis 1% vorliegen.
Den beschriebenen erfindungsgemäßen Mitteln lassen sich andere biozide Wirkstoffe beimischen. Es folgen einige Beispiele solcher Aufbereitungsformen oder Mittel.

## Granulat

Zur Herstellung eines 5%igen Granulates werden die folgenden Stoffe verwendet:

5 Teile eines der Wirkstoffe der Formel I,
0,25 Teile Epichlorhydrin,
0,25 Teile Cetylpolyglykoläther,
3,50 Teile Polyäthylenglykol,
91 Teile Kaolin (Korngröße: 0,3 – 0,8 mm).

Die Aktivsubstanz wird mit Epichlorhydrin vermischt und in 6 Teilen Aceton gelöst, hierauf wird Polyäthylenglykol und Cetylpolyglykoläther zugesetzt. Die so erhaltene Lösung wird auf Kaolin aufgesprüht und anschließend im Vakuum verdampft.

## Spritzpulver

Zur Herstellung eines a) 70%igen und b) 10%igen Spritzpulvers werden folgende Bestandteile verwendet:

a) 70 Teile 2-[4-(4'-Trifluormethyl-phenoxy)-phenoxy]-2-methoxy-essigsäuremethylester,
5 Teile Natriumdibutylnaphthylsulfonat,
3 Teile Naphthalinsulfonsäuren-Phenolsulfonsäuren-Formaldehyd-Kondensat 3 : 2 : 1,
10 Teile Kaolin,
12 Teile Champagne-Kreide;
b) 10 Teile 2-[4-(4'-Trifluormethylphenoxy)-phenoxy]-2-methoxy-essigsäuremethylester,
3 Teile Gemisch der Natriumsalze von gesättigten Fettalkoholsulfaten,
5 Teile Naphthalinsulfonsäure-Formaldehyd-Kondensat,
82 Teile Kaolin.

Der angegebene Wirkstoff wird auf die entsprechenden Trägerstoffe (Kaolin und Kreide) aufgezogen und anschließend mit den übrigen Bestandteilen vermischt und vermahlen. Man erhält Spritzpulver von vorzüglicher Benetzbarkeit und Schwebefähigkeit. Aus solchen Spritzpulvern können durch Verdünnen mit Wasser Suspensionen von 0,1 – 80% Wirkstoff erhalten werden, die sich zur Unkrautbekämpfung in Pflanzenkulturen eignen.

## Paste

Zur Herstellung einer 45%igen Paste werden folgende Stoffe verwendet:

45 Teile 2-[4-(4'-Trifluormethylphenoxy)-phenoxy]-2-methoxy-essigsäure-methylester oder eines anderen der genannten Wirkstoffe der Formel I,
5 Teile Natriumaluminiumsilikat,
14 Teile Cetylpolyglykoläther mit 8 Mol Äthylenoxid,
1 Teil Öylpolyglykoläther mit 5 Mol Äthylenoxid,
2 Teile Spindelöl,
10 Teile Polyäthenglykol,
23 Teile Wasser.

Der Wirkstoff wird mit den Zuschlagstoffen in dazu geeigneten Geräten innig vermischt und vermahlen. Man erhält eine Paste, aus der sich durch Verdünnen mit Wasser Suspensionen jeder gewünschten Konzentration herstellen lassen.

Emulsionskonzentrat

Zur Herstellung eines 25%igen Emulsionskonzentrates werden

25 Teile 2-[3-(3',5'-Dipyridyl-2'-oxy)-6-chlorphenoxy]-2-methoxy-essigsäuremethylester oder eines anderen der genannten Wirkstoffe der Formel I,
5 Teile einer Mischung von Nonylphenolpolyoxyäthylen oder Calciumdodecylbenzolsulfat,
15 Teile Cyclohexanon,
55 Teile Xylol

miteinander vermischt. Dieses Konzentrat kann mit Wasser zu Emulsionen auf geeignete Konzentrationen von z. B. 0,1 bis 10% verdünnt werden. Solche Emulsionen eignen sich zur Bekämpfung von Unkräutern in Kulturpflanzungen.

Erfindungsgemäße Mittel, die als aktive Komponente mindestens eine Verbindung der Formel I enthalten, eignen sich besonders zur selektiven Bekämpfung schwerbekämpfbarer monocotyler Ungräser in pre- und insbesondere post-emergenter Anwendung in Kulturpflanzenbeständen, wie z. B. Weizen, aber auch Soja, Baumwolle, Zuckerrohr etc.

Zum Nachweis der Brauchbarkeit als Herbizide (pre- und post-emergent) dienen folgende Testmethoden:

Pre-emergente Herbizid-Wirkung
(Keimhemmung)

Im Gewächshaus wird unmittelbar nach der Einsaat der Versuchspflanzen in Saatschalen die Erdoberfläche mit einer wäßrigen Dispersion der Wirkstoffe, erhalten aus einem 25%igen Emulsionskonzentrat resp. aus einem 25%igen Spritzpulver mit Wirkstoffen, die wegen ungenügender Löslichkeit nicht als Emulsionskonzentrat hergestellt werden können, behandelt. Es wurden vier verschiedene Konzentrationsreihen angewendet, entsprechend 4, 2, 1 und 0,5 kg Wirksubstanz pro Hektar. Die Saatschalen werden im Gewächshaus bei 22–25°C und 50–70% rel. Luftfeuchtigkeit gehalten und der Versuch nach 3 Wochen ausgewertet und die Resultate nach folgender Notenskala bonitiert:

1 = Pflanzen nicht gekeimt oder total abgestorben
2–3 = sehr starke Wirkung
4–6 = mittlere Wirkung
7–8 = geringe Wirkung
9 = keine Wirkung (wie unbehandelte Kontrolle)

als Versuchspflanzen dienen:

| | |
|---|---|
| hordeum (Gerste) | setaria italica |
| triticum (Weizen) | echinochloa crus galli |
| zea (Mais) | beta vulgaris |
| sorghum hybr. (Hirse) | sida spinosa |
| oryza (Reis) | sesbania exaltata |
| glycine (Soja) | amaranthus retroflexus |
| gossypium (Baumwolle) | sinapis alba |
| avena fatua | ipomoea purpurea |
| lolium perenne | galium aparine |
| alopecurus myosuroides | pastinaca sativa |
| bromus tectorum | rumex sp. |
| cyperus esculentus | chrysanthemum leucum. |
| rottboellia exaltata | abutilon sp. |
| digitaria sanguinalis | solanum nigrum |

Post-emergente Herbizid-Wirkung
(Kontaktherbizid)

Eine größere Anzahl (mindestens 7) Unkräuter und Kulturpflanzen, sowohl monocotyle wie dicotyle, wurde nach dem Auflaufen (im 4-bis-6-Blattstadium) mit einer wäßrigen Wirkstoffdispersion in Dosierungen von 0,06; 0,125; 0,25; 0,5; 1; 2 und 4 kg Wirksubstanz pro Hektar auf die Pflanzen gespritzt und diese bei 24°–26°C und 45–60% rel. Luftfeuchtigkeit gehalten. 15 Tage nach Behandlung wird der Versuch ausgewertet und das Ergebnis wie im pre-emergent-Versuch nach derselben Notenskala bonitiert.

Hemmung des vegetativen Wachstums in Sojapflanzen

In einem Sojabohnenfeld wurden Parzellen von je 30 auf 8 Fuß mit wäßrigen Lösungen der Wirkstoffe zu einem Zeitpunkt bespritzt, wo sich die Sojapflanzen im 9—10 Blatt-Stadium befanden. Unbehandelte Parzellen dienten als Kontrolle. Im Erntezeitpunkt, $3^1/_2$ Monate nach dem Applikationszeitpunkt, wurde die mittlere Wuchshöhe der Pflanzen in jeder Parzelle sowie die Erträge bestimmt und mit denen der unbehandelten Kontrollparzellen verglichen.

Defoliation und Dessikation in Baumwolle

In einem Baumwoll-Feld wurden 2 Wochen vor dem erwarteten Erntezeitpunkt Parzellen von je 2 Reihen auf 20 Fuß, mit wäßrigen Zubereitungen der Wirkstoffe bespritzt. Unbehandelte Parzellen dienten als Kontrollen. Am 3., 7. und 14. Tag nach der Applikation wurde der Blattfall und die Dessikationswirkung an den verschiedenen Parzellen bestimmt und mit dem Zustand der Kontrollparzellen verglichen.

**Patentansprüche**

1. Phenoxy-phenoxy-alkancarbonsäurederivate der Formel I

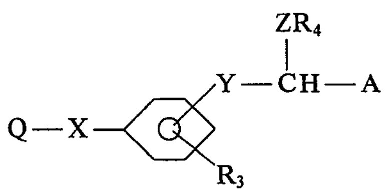

worin

Q ein Rest

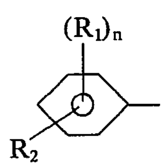

oder

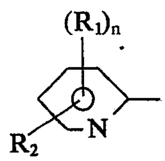

A die Cyanogruppe oder ein Rest-COB,
B ein Rest $-OR_5$, $-SR_6$, $-NR_7R_8$,
$R_1$ ein Halogenatom, n die Zahl 0, 1 oder 2,
$R_2$ ein Halogenatom oder der Trifluormethyl-, Nitro-, Cyano-, Carbamoyl- oder Thiocarbamoylrest,
$R_3$ Wasserstoff, Halogen, ein $C_1-C_4$ Alkyl-, der Nitro-, Cyano- oder Carbamoylrest,
$R_4$ $C_1-C_8$ Alkyl, $C_2-C_8$ Alkenyl, $C_2-C_8$ Alkinyl, Benzyl oder Phenyl gegebenenfalls durch Halogen substituiert, $C_2-C_8$ Alkoxyalkyl, $C_3-C_{12}$ Cycloalkyl,
$R_5$ Wasserstoff oder das Kation einer Base

$$\frac{1}{m} M^{m\oplus}$$

M ein Alkali-, Erdalkali-Kation oder ein Fe, Cu-, Zn-, Mn-, Ni-Kation oder einen Ammonio-Rest

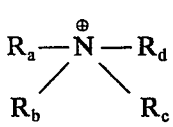

m als ganze Zahl 1, 2 oder 3 die Wertigkeit des Kations berücksichtigt, während $R_a$, $R_b$, $R_c$ und $R_d$ unabhängig voneinander Wasserstoff, Benzyl oder einen gegebenenfalls durch $-OH$, $-NH_2$ oder $C_1-C_4$ Alkoxy substituierten $C_1-C_4$ Alkylrest bedeuten, weiter bedeutet

$R_5$ und $R_6$ einen $C_1-C_{18}$ Alkyl-Rest, der unsubstituiert oder gegebenenfalls substituiert ist durch Halogen, Nitro, Cyano, $C_1-C_8$ Alkoxy, $C_2-C_8$-Alkoxyalkoxy; $C_3-C_6$ Alkenyloxy, $C_1-C_8$ Alkylthio, $C_2-C_8$ Alkanoyl, $C_2-C_8$ Acyloxy, $C_2-C_8$ Alkoxycarbonyl, Carbamoyl, Bis ($C_1-C_4$ alkyl)amino, Tris ($C_1-C_4$) alkyl)ammonio, $C_3-C_8$ Cycloalkyl, $C_3-C_8$ Cycloalkenyl, gegebenenfalls auch durch einen unsubstituierten oder seinerseits durch Halogen, $C_1-C_4$-Alkyl, $C_1-C_4$-Alkoxy ein- oder mehrfach substituierten Phenoxyrest oder 5-6-gliedrigen heterocyclischen Rest mit 1 bis 3 Heteroatomen;

- einen unsubst. oder ein- bis vierfach durch Halogen oder einmal durch Phenyl oder Methoxycarbonyl subst. $C_3-C_{18}$ Alkenylrest;
- einen $C_3-C_8$ Alkinyl-Rest;
- einen gegebenenfalls durch Halogen oder $C_1-C_4$-Alkyl substituierten $C_3-C_{12}$ Cycloalkyl-Rest;
- einen $C_3-C_8$ Cycloalkenyl-Rest;
- einen Phenylrest, der unsubstituiert oder durch Halogen, $C_1-C_4$-Alkyl, $C_1-C_4$-Alkoxy, $C_1-C_4$-Alkylthio, $NO_2$, $CF_3$, COOH, CN, OH, $SO_3H$, $NH_2$ oder $-NH(C_1-C_4$ Alkyl) oder $-N(C_1-C_4$ Alkyl$)_2$ ein- oder mehrfach substituiert ist;
- einen 5- bis 6gliedrigen heterocyclischen Ring mit 1 bis 3 Heteroatomen;

$R_7$ und $R_8$ je Wasserstoff oder einen niederen Alkylrest, oder eines davon auch einen verzweigten Alkenyl- oder Alkinylrest, oder den Phenyl-, Benzyl-, Cyclohexyl- oder Methoxyrest, oder

$R_7$ und $R_8$ können zusammen mit dem Stickstoffatom, an das sie gebunden sind, auch einen heterocyclischen Rest bilden,

X, Y und Z je ein Sauerstoff- oder Schwefelatom bedeuten.

2. Die Verbindungen gemäß Patentanspruch 1 der Formel

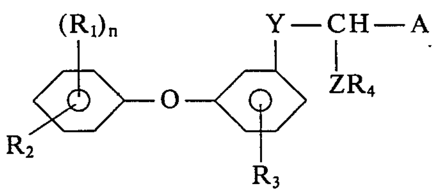

worin A, n, $R_1$, $R_2$, $R_3$, $R_4$, Y und Z die unter Formel I gegebene Bedeutung haben.

3. Die Verbindungen gemäß Patentanspruch 1 der Formel

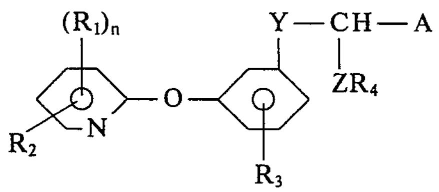

worin A, n, $R_1$, $R_2$, $R_3$, $R_4$, Y und Z die unter Formel I gegebene Bedeutung haben.

4. Als Verbindung gemäß Anspruch 1, 2-[3-(2'-Chlor-4'-trifluormethylphenoxy)-6-chlorphenoxy]-2-methoxy-essigsäure-methylester.

5. Als Verbindung gemäß Anspruch 1, 2-[3-(2'-Chlor-4'-trifluormethylphenoxy)-6-nitrophenoxy]-2-methoxy-essigsäure-methylester.

6. Als Verbindung gemäß Anspruch 1, 2-[3-(2'-Chlor-4'-trifluormethylphenoxy)-6-nitrophenoxy]-2-methylthio-essigsäure-methylester.

7. Als Verbindung gemäß Anspruch 1, 2-[3-(3',5'-Dichlorpyridyl-2'-oxy)-6-chlorphenoxy]-2-methoxy-essigsäure-methylester.

8. Verfahren zur Herstellung der Phenoxy-phenoxy-alkancarbonsäurederivate der Formel I, Anspruch 1, dadurch gekennzeichnet, daß man in an sich bekannter Weise in einem inerten Lösungsmittel in Gegenwart einer Base als säurebindendes Mittel ein entsprechend substituiertes Phenol oder Thiophenol der Formel II

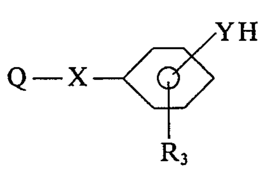

(II)

worin Q, $R_1$, n, $R_2$, $R_3$, X und Y die unter Formel I, Anspruch 1 gegebene Bedeutung haben, mit einem in 2-Stellung durch $-ZR_4$ substituierten Derivat einer 2-Halogenessigsäure der Formel III

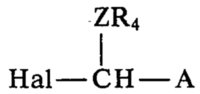

(III)

umsetzt, worin A und $R_4$ die im Anspruch 4 gegebene Bedeutung haben.

9. Verfahren zur Herstellung der Phenoxy-phenoxyalkancarbonsäurederivate der Formel I, Anspruch 1, dadurch gekennzeichnet, daß man in an sich bekannter Weise in einem inerten Lösungsmittel in Gegenwart einer Base als säurebindendes Mittel ein entsprechend substituiertes Derivat einer 2-Halogenessigsäure entsprechend der Formel IV .

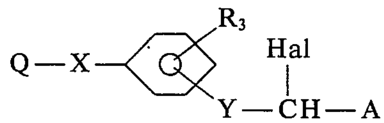

(IV)

worin A, Q, $R_3$, X und Y die unter Formel I, Anspruch 1 gegebene Bedeutung haben und »Hal« für ein Halogenatom steht, mit einem Alkohol oder Thiol der Formel V umsetzt,

$$H—Z—R_4 \qquad (V)$$

worin $R_4$ und Z die unter Formel I gegebene Bedeutung haben.

10. Herbizides und das Pflanzenwachstum regulierendes Mittel, dadurch gekennzeichnet, daß es als Wirkstoff mindestens ein Phenoxy-phenoxy-alkancarbonsäurederivat der Formel I, Anspruch 1 enthält.

11. Die Verwendung der Phenoxy-alkancarbonsäurederivate der Formel I, Anspruch 1 oder sie enthaltender Mittel zur selektiven Kontrolle von Unkräutern in Kulturen von Getreide, Reis.

12. Die Verwendung der Phenoxy-alkancarbonsäurederivate der Formel I, Anspruch 1 oder sie enthaltender Mittel zur Kontrolle des Unkrautes bromus tectorum.

13. Die Verwendung des Phenoxy-alkancarbonsäurederivate der Formel I, Anspruch I oder sie enthaltender Mittel zur Hemmung des vegetativen Wachstums in Sojakulturen.

14. Die Verwendung der Phenoxy-alkancarbonsäurederivate der Formel I, Anspruch 1 oder sie enthaltender Mittel zur Defoliation und Dessication von Kulturpflanzen, kurz bevor deren Ernte.

**Claims**

1. A phenoxy-phenoxy-alkanecarboxylic acid derivative of the formula I

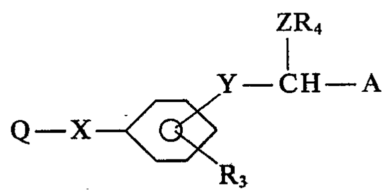

in which

Q is a radical

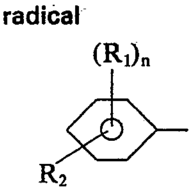

or

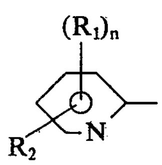

A is the cyano group or a radical $-COB$,
B is a radical $-OR_5$, $-SR_6$ or $-NR_7R_8$,
$R_1$ is a halogen atom,
n is the number 0, 1 or 2,
$R_2$ is a halogen atom or the trifluoromethyl, nitro, cyano, carbamoyl or thiocarbamoyl group,
$R_3$ is hydrogen, halogen, a $C_1-C_4$-alkyl group, or the nitro, cyano or carbamoyl group,
$R_4$ is $C_1-C_6$-alkyl, $C_2-C_6$-alkenyl, $C_2-C_6$-alkynyl, benzyl or phenyl both optionally substituted by halogen, or it is $C_2-C_6$-alkoxyalkyl or $C_3-C_{12}$-cycloalkyl,
$R_5$ is hydrogen or the cation of a base $\frac{1}{m} M^{m\oplus}$ wherein
M is an alkali metal cation or an alkaline-earth metal cation or an Fe, Cu, Zn, Mn or Ni cation, or an ammonium group

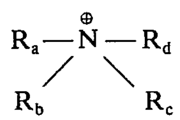

and
m as integer 1, 2 or 3 takes account of the valency of the cation, and $R_a$, $R_b$, $R_c$ and $R_d$ independently of one another are each hydrogen, benzyl or a $C_1-C_4$-alkyl group which is unsubstituted or substituted by $-OH$, $-NH_2$ or $C_1-C_4$-alkoxy,
$R_5$ and $R_6$ are each a $C_1-C_{18}$-alkyl group which is unsubstitued or is substituted by halogen, nitro, cyano, $C_1-C_8$-alkoxy, $C_2-C_8$-alkoxyalkoxy, or by $C_3-C_6$-alkenyloxy, $C_1-C_8$-alkylthio, $C_2-C_8$-alkanoyl, $C_2-C_8$-acyloxy, $C_2-C_8$-alkoxycarbonyl, carbamoyl, or by bis-($C_1-C_4$-alkyl)-amino, tris-($C_1-C_4$-alkyl)-ammonium, $C_3-C_8$-cycloalkyl or $C_3-C_8$-cycloalkenyl, optionally also by a phenoxy group or a 5–6-menbered heterocyclic radical having 1 to 3 hetero atoms, each of which is unsubstituted or in its turn mono- or polysubstituted by halogen, $C_1-C_4$-alkyl or $C_1-C_4$-alkoxy; or $R_5$ and $R_6$ are each a $C_3-C_{18}$-alkenyl group which is unsubstituted or is mono- to tetrasubstituted by halogen or monosubstituted by phenyl or methoxycarbonyl; or they are each a $C_3-C_8$-alkynyl group; or a $C_3-C_{12}$-cycloalkyl group which is unsubstituted or substituted by halogen or $C_1-C_4$-alkyl; or they are each a $C_3-C_8$-cycloalkenyl group; or a phenyl group which is unsubstituted or is mono- or polysubstituted by halogen, $C_1-C_4$-alkyl, $C_1-C_4$-alkoxy, $C_1-C_4$-alkylthio, $NO_2$, $CF_3$, COOH, CN, OH, $SO_3H$, $NH_2$, or $-NH(C_1-C_4\text{-alkyl})$ or $-N(C_1-C_4\text{-alkyl})_2$; or $R_5$ and $R_6$ are each a 5- to 6-membered heterocyclic ring having 1 to 3 hetero atoms; and
$R_7$ and $R_8$ are each hydrogen or a lower alkyl group, or one of them can also be a branched-chain alkenyl or alkynyl group, or the phenyl, benzyl, cyclohexyl or methoxy group, or
$R_7$ and $R_8$ together with the nitrogen atom to which they are attached can also form a heterocyclic ring, and
X, Y and Z are each an oxygen atom or a sulfur atom.

2. A compound according to Claim 1 of the formula

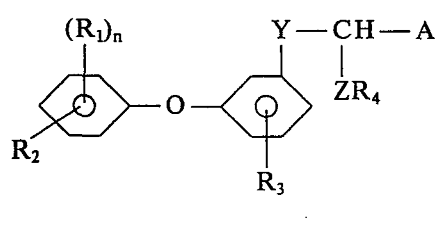

in which A, n, $R_1$, $R_2$, $R_3$, $R_4$, Y and Z have the meanings given under the formula I.

3. A compound according to Claim 1 of the formula

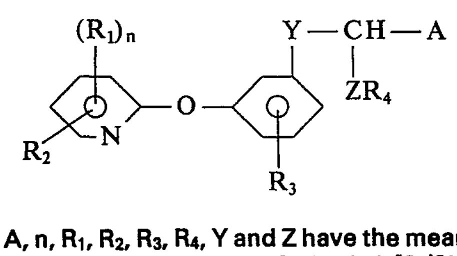

in which A, n, $R_1$, $R_2$, $R_3$, $R_4$, Y and Z have the meanings given under the formula I.

4. As compound according to Claim 1, 2-[3-(2'-chloro-4'-trifluoromethylphenoxy)-6-chlorophenoxy]-2-methoxyacetic acid methyl ester.

5. As compound according to Claim 1, 2-[3-(2′-chloro-4′-trifluoromethylphenoxy)-6-nitrophenoxy]-2-methoxyacetic acid methyl ester.

6. As compound according to Claim 1, 2-[3-(2′-chloro-4′-trifluoromethylphenoxy)-6-nitrophenoxy]-2-methylthioacetic acid methyl ester.

7. As compound according to Claim 1, 2-[3-(3′,5′-dichloropyridyl-2′-oxy)-6-chlorophenoxy-2-methoxyacetic acid methyl ester.

8. A process for producing the phenoxy-phenoxy-alkanecarboxylic acid derivative of the formula I, Claim I, which process comprises reacting, in a manner known per se, an appropriately substituted phenol or thiophenol of the formula II

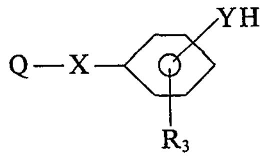

(II)

in which Q, $R_3$, X and Y have the meanings given under the formula I, Claim 1, in an inert solvent, and in the presence of a base as acid-binding agent, with a derivative, substituted in the 2-position by $-ZR_4$, of a 2-haloacetic acid of the formula III

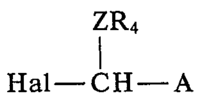

(III)

in which A and $R_4$ have the meanings given in Claim 1.

9. A process for producing the phenoxy-phenoxy-alkanecarboxylic acid derivative of the formula I, Claim 1, which process comprises reacting, in a manner known per se, an appropriately substituted derivative of a 2-haloacetic acid corresponding to the formula IV

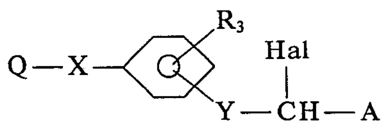

(IV)

in which A, Q, $R_3$, X and Y have the meanings given under the formula I, Claim 1, and »Hal« is a halogen atom, in an inert solvent and in the presence of a base as acidbinding agent, with an alcohol or thiol of the formula V

$$H—Z—R_4 \qquad (V)$$

in which $R_4$ and Z have the meanings given under the formula I.

10. A herbicidal and plant-growth-regulating composition which contains as active substance at least one phenoxy-phenoxy-alkanecarboxylic acid derivative of the formula I, Claim 1.

11. The use of a phenoxy-alkanecarboxylic acid derivative of the formula I, Claim 1, or of a composition containing ist, for the selective control of weeds in crops of cereals or rice.

12. The use of a phenoxy-alkanecarboxylic acid derivative of the formula I, Claim 1, or of a composition containing it, for the control of the weed bromus tectorum.

13. The use of a phenoxy-alkanecarboxylic acid derivative of the formula I, Claim 1, or of a composition containing it, for reducing the vegetative growth in soya-bean crops.

14. The use of a phenoxy-alkanecarboxylic acid derivative of the formula I, Claim 1, or of a composition containing it, for defoliation and desiccation of cultivated plants shortly before the harvesting thereof.

**Revendications**

1. Dérivés d'acides phénoxy-alcoyl-carboxyliques de formule I

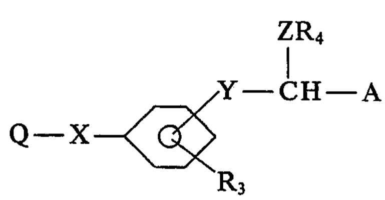

où

Q représente un radical

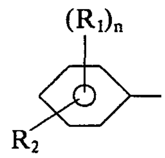

ou

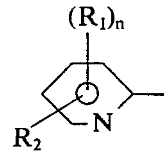

A le groupe cyano ou un radical $-COB$,
B un radical $-OR_5$, $-SR_6$, $-NR_7R_8$,
$R_1$ un atome d'halogène, n le nombre 0, 1 ou 2,
$R_2$ un atome d'halogène ou un radical trifluorométhyle, nitro, cyano, carbamoyle ou thiocarbamoyle,
$R_3$ un hydrogène, un halogène, un alcoyle en $C_1$ à $C_4$, un radical nitro, cyano ou carbamoyle,
$R_4$ un alcoyle en $C_1$ à $C_6$, un alcényle en $C_2$ à $C_6$, un alcynyle en $C_2$ à $C_6$, un benzyle ou un phényle, éventuellement substitué par un halogène, un alcoxyalcole en $C_2$ à $C_6$, un cycloalcoyle en $C_3$ à $C_{12}$,
$R_5$ un hydrogène ou le cation d'une base

$$\frac{1}{m} M^{m\oplus}$$

M un cation alcalin, alcalino-terreux ou un cation Fe, Cu, Zn, Mn, Ni ou un radical ammonio

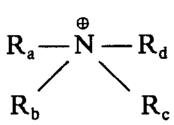

m en tant que nombre entier 1, 2 ou 3 tient compte de la valence du cation, tandis que $R_a$, $R_b$, $R_c$ et $R_d$ représentent indépendamment l'un de l'autre un hydrogène, un benzyle ou un radical alcoyle en $C_1$ à $C_4$ éventuellement substitué par $-OH$, $-NH_2$ ou un alcoxy en $C_1$ à $C_4$, et en outre

$R_5$ et $R_6$ représentent un radical alcoyle en $C_1$ à $C_{18}$, qui est non substitué ou éventuellement substitué par un halogène, un nitro, un cyano, un alcoxy en $C_1$ à $C_8$, un alcoxyalcoxy en $C_2$ à $C_8$, un alcényloxy en $C_3$ à $C_6$, un alcoylthio en $C_1$ à $C_8$, un alcanoyle en $C_2$ à $C_8$, un acyloxy en $C_2$ à $C_8$, un alcoxycarbonyle en $C_2$ à $C_8$, un carbamoyle; un bis-alcoyle en $C_1$ à $C_4$)amino, un tris(alcoyle en $C_1$ à $C_4$)ammonio, un cycloalcoyle en $C_3$ à $C_8$, un cycloalcényle en $C_3$ à $C_8$, éventuellement également radical phénoxy ou un radical hétérocyclique à 5 ou 6 chaînons ayant de 1à 3 hétéroatomes mono- ou polysubstitué également mono- ou poly-substitué, un radical éventuellement aussi substitué par un radical phénoxy non substitué ou mono- ou poly-substitué de son côté par un halogène, un alcoyle en $C_1$ à $C_4$ ou un alcoxy en $C_1$ à $C_4$ ou un radical hétérocyclique à 5 ou 6 chainons ayant de 1à 3 hétéroatomes;

- un radical alcényle en $C_3$ à $C_{18}$ non substitué ou mono- à tétra-substitué par un halogène ou monosubstitué par un phényle ou un méthoxy-carbonyle;
- un radical alcynyle $C_3$ à $C_8$;
- un radical cycloalcoyle en $C_3$ à $C_{12}$ éventuellement substitué par un halogène ou un alcoyle en $C_1$ à $C_4$;
- un radical cycloalcényle en $C_3$ à $C_8$;
- un radical phényle, qui est non substitué ou mono- ou polysubstitué par un halogène, un alcoyle en $C_1$ à $C_4$, un alcoxy en $C_1$ à $C_4$, un alcoylthio en $C_1$ à $C_4$, $NO_2$, $CF_3$, COOH, CN, OH, $SO_3F$, $NH_2$ ou $-NH$(alcoyle en $C_1$ à $C_4$) ou $-N$(alcoyle en $C_1$ à $C_4)_2$;
- un noyau hétérocyclique à 5 à 6 chainons ayant de 1 à 3 hétéroatomes;

$R_7$ et $R_8$ représentent chacun un hydrogène ou un radical alcoyle inférieur, ou encore l'un d'entre représente un radical alcényle ou alcynyle ou le radical phényle, benzyle, cyclohexyle ou méthoxy,
$R_7$ et $R_8$ forment également ensemble, avec l'atome d'azote auquel ils sont liés, un radical hétérocyclique,

X, Y et Z représentent chacun un atome d'oxygène ou de soufre.

2. Composés selon la revendication 1 de formule

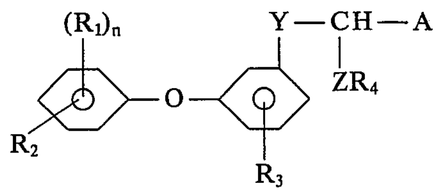

où A, n, $R_1$, $R_2$, $R_3$, $R_4$, Y et Z ont la signification donnée pour la formule I.

3. Composés selon la revendication 1 de formule

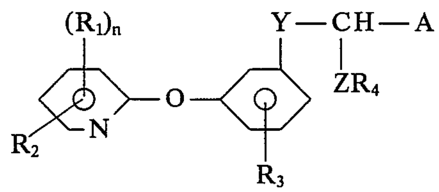

où A, n, $R_1$, $R_2$, $R_3$, $R_4$, Y et Z ont la signification donnée pour la formule I.

4. Comme composé selon la revendication 1, l'ester méthylique de l'acide 2-[3-(2'-Chloro-4'-trifluorométhylphénoxy)-6-chlorophényoxy]-2-méthoxy acétique.

5. Comme composé selon la revendication 1, l'ester méthylique de l'acide 2-[3-(2'-Chloro-4'-trifluorométhylphénoxy)-6-nitrophénoxy]-2-méthoxy-acétique.

6. Comme composé selon la revendication 1, l'ester méthylique de l'acide 2-[3-(2'-Chloro-4'-trifluorométhylphénoxy)-6-nitrophénoxy]-2-méthylthio-acétique.

7. Comme composé selon la revendication 1, l'ester méthylique de l'acide 2-[3-(3',5'-Dichloropyridyl-2'-oxy)-6-chlorophénoxy]-2-méthoxy-acétique.

8. Procédé de préparation des dérivés d'acides phénoxy-phénoxy-alcoyl-carboxyliques de formule I de la revendication 1, caractérisé en ce qu'on fait réagir de façon classique dans un solvant en présence d'une base comme liant acide un phénol ou thiophénol de formule II substitué de façon correspondante

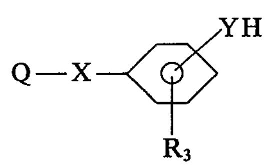

(II)

où Q, $R_3$, X et Y ont la signification donnée pour la formule I de la revendication 1, avec un dérivé, substitué en position 2 par $-ZR_4$, d'un acide 2-halogènacétique de formule III

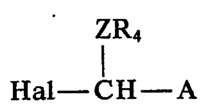

(III)

où A et $R_4$ ont la signification donnée dans la revendication 1.

9. Procédé de préparation des dérivés d'acides phénoxy-phénoxy-alcoyl-carboxyliques de formule I de la revendication 1, caractérisé en ce qu'on fait réagir de façon classique dans un solvant inerte en présence d'une base comme liant acide un dérivé, substitué de façon correspondante, d'un acide 2-halogènacétique correspondant à la formule IV

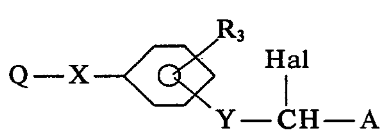

(IV)

où A, Q, $R_3$, X et Y ont la signification donnée pour la formule I de la revendication 1 et où »Hal« représente un atome d'halogène, avec un alcool ou un thiol de formule V

$$H—Z—R_4 \qquad (V)$$

où $R_4$ et Z ont la signification donnée pour la formule I.

10. Agent herbicide et régulateur de la croissance des plantes, caractérisé en ce qu'il contient comme matière active au moins un dérivé d'acide phénoxy-phénoxy-alcoyl-carboxylique de formule I de la revendication 1.

11. Application des dérivés d'acide phénoxy-alcoyl-carboxylique de formule I de la revendication 1 ou des agents qui les contiennent à la lutte sélective contre les mauvaises herbes dans les cultures de céréales et de riz.

12. Application des dérivés d'acides phénoxy-alcoyl-carboxyliques de formule I de la revendication 1 ou des agents qui les contiennent à la lutte contre la mauvaise herbe bromus tectorum.

13. Application des dérivés d'acides phénoxy-alcane-carboxyliques de formule I de la revendication 1 ou des agents qui les contiennent à l'inhibition de la croissance végétative des cultures de soja.

14. Application des dérivés d'acides phénoxy-alcoyl-carboxyliques de formule I de la revendication 1 ou des agents qui les contiennent à la défoliation et à la dessication des plantes cultivées peu avant leur récolte.